# FASCICULE DE BREVET EUROPEEN

(11) **EP 0 837 843 B1**
(45) Date de publication et mention de la délivrance du brevet: **12.09.2001**
(21) Numéro de dépôt: 96922085.4
(22) Date de dépôt: 10.06.1996
(51) Int. Cl.: C07C 229/12, C07C 227/14

(54) **PROCEDE DE PREPARATION DES FORMES ENANTIOMERES DE L'ACIDE AMINO ALKYLAMINOPHENYL PROPANOIQUE**
VERFAHREN ZUR HERSTELLUNG VON ENANTIOMEREN DER AMINOALKYLAMINOPHENYLPROPANSÄURE
METHOD FOR PREPARING ENANTIOMERIC FORMS OF AMINO ALKYLAMINOPHENYL PROPANOIC ACID

(30) Priorité: 12.06.1995 FR 9506890
(43) Date de publication de la demande: 29.04.1998
(73) Titulaire: Aventis Pharma S.A., 92160 Antony (FR)
(72) Inventeur: STAMMLER, Robert, F-75012 Paris (FR)
(74) Mandataire: Le Pennec, Magali
(86) Numéro de dépôt international: FR9600872
(87) Numéro de publication internationale: WO9641794

(56) Documents cités:
- CHEMICAL ABSTRACTS, vol. 55, no. 22, 1961 Columbus, Ohio, US; abstract no. 22226g, B.L. MOLDAVER ET AL.: "Synthesis of some derivatives of beta-phenyl-DL-alanine" XP002012679 cité dans la demande & ZHUR. OBSHNEI KHIM., vol. 31, 1961, pages 1560-1569,
- CHEMICAL ABSTRACTS, vol. 53, no. 13, 1959 Columbus, Ohio, US; abstract no. 12202g, F. BERGEL ET AL.: "Cytoactive amino acids and peptides. V. Derivatives of para-amino- and para-mercaptophenylalanine" XP002012680 & J. CHEM. SOC., 1959, pages 90-97,

## Description

La présente invention concerne un procédé de préparation d'une forme énantiomère de l'acide amino-2 (alkylamino-4 phényl)-3 propanoïque de formule générale : 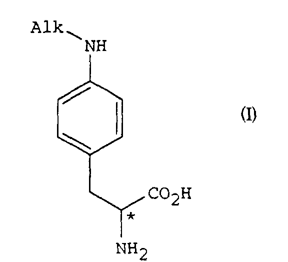 dans laquelle Alk représente un radical alcoyle contenant 1 à 2 atomes de carbone et ses sels.

La préparation de l'acide amino-2 (méthylamino-4 phényl)-3 propanoïque (2S) a été décrite dans la demande de brevet WO 94/08014 par action d'une N-méthyltransférase sur la p.amino-(L)-phénylalanine. Cependant elle ne serait pas exploitable industriellement en raison des rendements très faibles.

La préparation d'acide amino-2 (méthylamino-4 phényl)-3 propanoïque racémique a été décrite par B.L. MOLDAVER et Z.V. PUSHKAREVA, Chem. Abstr., 55, 22226g ; de plus la spécificité de cette réaction ne garantit pas la production de composé N-monométhylé (en synthétisant le diéthyl p-diméthylaminobenzyl-N-acétylaminomalonate, on observe, après cristallisation, la présence d'un mélange de p-aminophényl-alanine et de ses dérivés mono et diméthylé dans les eaux mères de cristallisation). La préparation d'acide amino-2 (méthylamino-4 phényl)-3 propanoïque racémique a également été décrite dans Chem. Abstr., 53(13), 12202g.

Il a été maintenant trouvé que les formes énantiomères de cet acide peuvent être préparées directement à partir de la (L)-phénylalanine ou de la (D)-phénylalanine, selon que l'on souhaite obtenir respectivement la forme (S) ou (R) de l'acide, avec des rendements élevés et une bonne qualité de produit.

Selon l'invention la (L) ou la (D)-phénylalanine, dont la fonction amine est éventuellement protégée, est nitrée, puis le radical nitro de la nitro-4 phénylalanine obtenue, de formule générale : 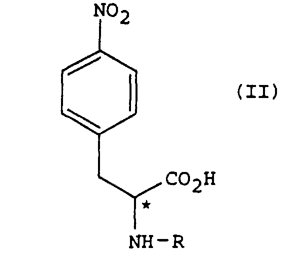 dans laquelle R est un atome d'hydrogène ou un radical protecteur, est transformé en un radical alcoylamino, après protection (si R est hydrogène), de la fonction amine de la phénylalanine.

La nitration s'effectue par l'acide nitrique en milieu sulfurique, à une température comprise entre -10 et -20°C.

La protection du radical amino s'effectue selon les méthodes connues, par un radical protecteur R dont la mise en place et l'élimination n'altère pas le reste de la molécule. Notamment, on opère selon les méthodes décrites par T.W. GREENE, Protective Groups in Organic Synthesis, A. Wiley-Interscience Publication (1981), ou par Mc OMIE, Protective Groups in Organic Chemistry, Plenum Press (1973). De préférence le radical amino est protégé à l'état d'amide par un radical R = acyle, plus particulièrement par un radical acétyle ; ou bien le radical amino est protégé à l'état de carbamate par un radical R = alcoyloxycarbonyle.

La transformation du radical nitro en un radical alcoylamino s'effectue par réduction alkylante lorsque le radical Alk est un radical méthyle ou éthyle, ou par réduction, formylation de l'amine obtenue et réduction subséquente du radical formylamino lorsque le radical Alk représente méthyle.

La réduction alkylante est mise en oeuvre par hydrogénation, sous pression d'hydrogène en présence de nickel de Raney, en opérant dans le méthanol à une température comprise entre 20 et 40°C et sous une pression comprise entre 100 et 200 kPa.
L'alkylation est effectuée sous pression d'hydrogène par addition de benzaldéhyde puis de formaldéhyde dans le cas où Alk est un radical méthyle ou d'éthanal dans le cas où Alk est un radical éthyle.
L'élimination du radical protecteur de l'amino et l'élimination du radical benzyle s'effectuent successivement. Lorsque le radical protecteur de l'amino est un radical acétyle, l'élimination s'effectue par traitement en milieu acide aqueux, notamment par l'acide chlorhydrique. Lorsque Alk est un radical méthyle ou éthyle, l'élimination du radical benzyle s'effectue par hydrogénation sous hydrogène en présence de palladium sur charbon en milieu acide aqueux, notamment l'acide chlorhydrique aqueux à une température comprise entre 50 et 60°C et sous une pression comprise entre 100 et 200 kPa. Dans ce cas le dérivé de phénylalanine de formule générale (I) est obtenu sous forme de sel. Eventuellement il peut être libéré de son sel selon les méthodes habituelles qui n'altèrent pas le reste de la molécule.

Lorsque l'on veut obtenir un dérivé de phénylalanine de formule générale (I) pour lequel Alk est méthyle par réduction puis formylation, la réduction du radical nitro s'effectue sur la nitro-4 phénylalanine de formule générale (II) dont la fonction amine est protégée par un radical R et dont la fonction acide a été préalablement protégée, en opérant en milieu réducteur comme par exemple par traitement par le zinc et le chlorure d'ammonium dans un mélange méthanol-eau. La formylation de l'amine obtenue s'effectue par l'acide formique, selon les méthodes habituelles de réaction d'un acide sur une amine, qui n'altèrent pas le reste de la molécule. Notamment on opère dans le tétrahydrofuranne à une température comprise entre 0 et 25°C. La réduction du radical formylamino ainsi formé est effectuée avantageusement par les boranes. Par exemple on opère au moyen de borane methyl sulfure dans le tétrahydrofuranne, à une température inférieure à 25°C.

La protection de l'amine s'effectue comme décrit précédemment. La protection de la fonction acide carboxylique s'effectue selon les méthodes connues qui n'altèrent pas le reste de la molécule, notamment par estérification. L'élimination du radical protecteur de l'amine et du radical protecteur de l'acide sont effectuées simultanément par traitement en milieu acide aqueux notamment par l'acide chlorhydrique.

Le produit de formule générale (I) peut être éventuellement purifié par des méthodes physiques telles que la cristallisation ou la chromatographie.

Le produit préparé selon l'invention peut être soit obtenu directement sous forme de sel soit transformé en sel d'addition avec les acides, en sel métallique ou en sel d'addition avec les bases azotées selon les méthodes connues en soi. Ces sels peuvent être obtenus par exemple par action d'une base métallique (par exemple alcaline ou alcalino-terreuse), de l'ammoniac ou d'une amine sur un produit selon l'invention dans un solvant approprié tel qu'un alcool, un éther ou l'eau ou par réaction d'échange avec un sel d'un acide organique. Le sel formé précipite après concentration éventuelle de sa solution, il est séparé par filtration, décantation ou lyophilisation.

Comme exemples de sels, peuvent être cités les sels avec les métaux alcalins (sodium, potassium, lithium) ou avec les métaux alcalino-terreux (magnésium, calcium), le sel d'ammonium, les sels de bases azotées (éthanolamine, diéthanolamine, triméthylamine, triéthylamine, méthylamine, propylamine, diisopropylamine, NN-diméthyléthanolamine, benzylamine, dicyclohexylamine, N-benzyl phénéthylamine, NN'-dibenzyléthylènediamine, diphénylènediamine, benzhydrylamine, quinine, choline, arginine, lysine, leucine, dibenzylamine), ainsi que les sels d'addition avec des acides minéraux (chlorhydrates, bromhydrates, sulfates, nitrates, phosphates) ou organiques (succinates, fumarates, maléates, méthanesulfonates, p.toluènesulfonates, iséthionates, tartrates, acétates, propionates, citrates, ou avec des dérivés de substitution de ces composés).

La forme énantiomère de l'acide amino-2 (alkylamino-4 phényl)-3 propanoïque ainsi préparée par le procédé selon l'invention peut être utile en synthèse chimique. Notamment elle peut être utilisée dans la préparation de produits biologiquement actifs et plus particulièrement de dérivés de synergistines comme la pristinamycine IB ou le dérivé éthylamino correspondant.

Les exemples suivants donnés à titre non limitatif illustrent l'invention.

### Exemple 1

A une suspension de 200 g de (L)-phénylalanine dans 1660 cm³ d'eau à 20°C sont ajoutés, sous agitation, 340 cm³ de triéthylamine. Le mélange est maintenu sous agitation à une température voisine de 20°C pendant 30 minutes jusqu'à la solubilisation complète puis refroidi à 5°C. A cette solution sont additionnés 126 cm³ d'anhydride acétique goutte à goutte en conservant une température interne voisine de 5°C. En fin d'addition, le mélange est maintenu à 5°C pendant une heure puis refroidi à 0°C. A cette solution sont ajoutés goutte à goutte 200 cm³ d'une solution d'acide chlorhydrique à 36 % en maintenant la température voisine de 0°C. En fin d'addition la suspension est agitée une heure à 0°C, puis filtrée. Le précipité blanc obtenu est rincé par 400 cm³ d'eau, essoré, puis séché sous pression réduite (20 kPa) à environ 50°C.

On obtient ainsi 227 g d'acide acétamido-2 phényl-3 propanoïque (2S) sous forme d'un solide blanc fondant à 160°C.

### Préparation de l'acide acétamido-2 (nitro-4 phényl)-3 propanoïque (2S) :

A une solution agitée de 32,0 g d'acide acétamido-2 phényl-3 propanoïque (2S) dans 46,1 cm³ d'acide sulfurique à 90 % et refroidie à -15°C, sont ajoutés goutte à goutte 6,4 cm³ d'acide nitrique à 100 % en maintenant une température du mélange voisine de -15°C. A la fin de l'addition, la solution est maintenue à -15°C pendant une heure supplémentaire puis portée à 40°C, température à laquelle sont ajoutés 70,4 cm³ d'eau. En fin d'addition d'eau on laisse la solution revenir à une température voisine de 20°C. Le précipité obtenu est filtré, rincé par 2 fois 32 cm³ d'eau, essoré puis séché sous pression réduite (20 kPa) à environ 50°C.

On obtient ainsi 18,2 g d'acide acétamido-2 (nitro-4 phényl)-3 propanoïque (2S) sous forme d'un solide blanc se décomposant à 191°C.

### Préparation du dichlorhydrate de l'acide acétamido-2 ((N-méthylbenzylamino)-4 phényl)-3 propanoïque (2S) :

A 5,50 g de nickel Raney est ajoutée, à 20°C sous agitation, une solution de 5,00 g d'acide acétamido-2 (nitro-4 phényl)-3 propanoïque (2S) dans 100 cm³ de méthanol. La solution ainsi obtenue est purgée trois fois à l'azote puis trois fois à l'hydrogène avant d'être placée sous une pression d'hydrogène de 100 kPa sous agitation vigoureuse à une température voisine de 30°C pendant 2,5 heures. A la solution ramenée à 25°C sont additionnés goutte à goutte 2,90 cm³ de benzaldéhyde. Le mélange est maintenu sous agitation et sous pression d'hydrogène de 100 kPa pendant 2 heures puis 1,65 cm³ d'une solution aqueuse de formaldéhyde à 35 % y sont ajoutés. L'agitation sous pression d'hydrogène est poursuivie encore 2 heures puis la solution est purgée à l'azote, filtrée sur clarcel avant d'être concentrée à sec sous pression réduite (20 kPa) à une température voisine de 40°C.

On obtient ainsi 6,06 g d'acide acétamido-2 ((N-méthylbenzylamino)-4 phényl)-3 propanoïque (2S) sous forme d'un solide beige se décomposant à 160°C.

### Préparation du dichlorhydrate de l'acide amino-2 ((N-méthylamino)-4 phényl)-3 propanoïque (25) :

A une suspension de 1,60 g d'acide acétamido-2 ((N-méthyl-benzylamino)-4 phényl)-3 propanoïque (2S) dans 4,80 cm³ d'eau à 20°C sont ajoutés sous agitation 9,60 cm³ d'une solution aqueuse d'acide chlorhydrique à 36 %. La solution ainsi obtenue est portée à reflux pendant 2,5 heures, ramenée à 20°C, puis coulée sur une solution de 0,16 g de palladium sur charbon à 5 % dans 1,60 cm³ d'eau. Le mélange ainsi obtenu est purgé trois fois à l'azote puis trois fois à l'hydrogène avant d'être placé sous une pression d'hydrogène de 100 kPa sous agitation vigoureuse à une température voisine de 50°C pendant 2 heures puis ramené à 20°C, filtré sur clarcel, rincé par 2 fois 5,00 cm³ d'eau avant d'être concentré à sec sous pression réduite (20 kPa) à une température voisine de 40°C. Sur l'extrait sec sont ajoutés 12,90 cm³ de propanol-2. La suspension ainsi obtenue est portée à reflux pendant 1 heure, ramenée à une température voisine de 20°C, filtrée, rincée par 3,00 cm³ de propanol-2, essorée puis séchée sous pression réduite (20 kPa) à environ 40°C.

On obtient ainsi 1,11 g du dichlorhydrate de l'acide amino-2 ((N-méthylamino)-4 phényl)-3 propanoïque (2S) sous forme d'un solide beige se décomposant à 208°C.

[ee = 96,5 % par chromatographie en phase liquide sur une phase stationnaire chirale composée d'un éther couronne imprégné sur silice (CROWNPAK CR® )].

### Exemple 2

Préparation de l'acide amino-2 (nitro-4 phényl)-3 propanoïque (2S)

A une solution agitée de 33,37 g de (L)-phénylalanine dans 56,1 cm³ d'acide sulfurique à 95 % et refroidie à -15°C, sont ajoutés goutte à goutte 9,95 cm³ d'acide nitrique à 100 % en maintenant une température du mélange voisine de -16°C. A la fin de l'addition, la solution est maintenue à -16°C pendant 2,5 heures, 250 cm³ d'eau y sont ajoutés goutte à goutte en laissant la solution revenir à une température voisine de -10°C, puis 180 cm³ environ d'une solution aqueuse de soude à 30% y sont ajoutés goutte à goutte jusqu'à pH = 4,70 en laissant la solution revenir à une température voisine de 25°C. Le précipité obtenu est filtré, repris par 350 cm³ d'eau, porté à une température voisine de 50°C pendant 0,5 heure, ramené à une température voisine de 20°C, filtré, essoré puis séché sous pression réduite (20 kPa) à environ 40°C.

On obtient ainsi 24,35 g d'acide amino-2 (nitro-4 phényl)-3 propanoïque (2S) sous forme d'un solide blanc se décomposant à 230°C.

### Préparation de l'acide acétamido-2 (nitro-4 phényl)-3 propanoïque (2S) :

A une suspension de 19,9 g d'acide amino-2 (nitro-4 phényl)-3 propanoïque (2S) dans 78,6 cm³ d'eau à 20°C sont ajoutés, sous agitation , 20,9 cm³ d'une solution aqueuse de soude à 30 %. Le mélange est maintenu sous agitation à une température voisine de 20°C pendant 30 minutes puis refroidi à 5°C. A cette solution sont additionnés 13,7 cm³ d'anhydride acétique goutte à goutte en conservant une température interne voisine de 5°C. En fin d'addition, le mélange est maintenu à 5°C pendant 0,25 heure puis 9,5 cm³ d'une solution aqueuse de soude à 30 % sont ajoutés jusqu'à une complète solubilisation. A cette solution sont ajoutés goutte à goutte 26,0 cm³ d'une solution d'acide chlorhydrique à 36 % en laissant la température remonter à 15°C. En fin d'addition la suspension est agitée 0,5 heure à 15°C, filtrée, essorée, puis séchée sous pression réduite (20 kPa) à environ 40°C.

On obtient ainsi 19,5 g d'acide acétamido-2 (nitro-4 phényl)-3 propanoïque (2S) sous forme d'un solide blanc se décomposant à 191°C.

L'acide amino-2 [(N-méthylamino)-4 phényl]-3 propanoïque (2S) peut être obtenu à partir de l'acide acétamido-2 (nitro-4 phényl)-3 propanoïque (2S), comme décrit à l'exemple 1.

### Exemple 3

Préparation du chlorhydrate de l'amino-2 (nitro-4 phényl)-3 propanoate de méthyle (2S):

A une suspension de 177,4 g d'acide amino-2 (nitro-4 phényl)-3 propanoïque (2S) dans 1800 cm³ de méthanol à 0°C sont ajoutés goutte à goutte, sous agitation, 69,8 cm³ de chlorure de thionyle en maintenant la température du mélange entre 0 et 10°C. En fin d'addition le mélange est porté à reflux pendant 6 heures. Sur le mélange à reflux sont ensuite additionnés 900 cm³ de propanol-2 puis distillés 1600 cm³ de méthanol. La suspension ainsi obtenue est ramenée à température ambiante, maintenue une heure à l'ambiante sous agitation, filtrée, rincée par 180 cm³ de propanol-2, essorée puis séchée sous pression réduite (20 kPa) à environ 40°C.

On obtient ainsi 188,1 g de chlorhydrate de l'amino-2 (nitro-4 phényl)-3 propanoate de méthyle (2S) sous forme d'un solide blanc fondant à 218°C.

### Préparation de l'éthanamido-2 (nitro-4 phényl)-3 propanoate de méthyle (2S) :

A une suspension de 186,1 g de chlorhydrate de l'amino-2 (nitro-4 phényl)-3 propanoate de méthyle (2S) dans 930 cm³ de tétrahydrofuranne à 20°C sous agitation sont ajoutés 83 cm³ d'anhydride acétique. Le mélange est ramené à 5°C puis 241 cm³ de triéthylamine sont additionnés goutte à goutte de telle sorte que la température du mélange réactionnel soit maintenue inférieure à 20°C. En fin d'addition le mélange est maintenu sous agitation à 20°C pendant une heure puis refroidi à 5°C. A 5°C sont additionnés 460 cm³ d'eau. Le mélange est ramené à 20°C, agité pendant 30 minutes à cette température puis décanté. La phase inférieure aqueuse est soutirée et la phase supérieure organique est coulée sur 2300 cm³ d'eau à 20°C. Le mélange est porté à reflux et 600 cm³ de tétrahydrofuranne sont distillés. La suspension obtenue est ramenée à 20°C sous agitation, filtrée, rincée trois fois par 200 cm³ d'eau, essorée puis séchée sous pression réduite (20 kPa) à environ 50°C.

On obtient ainsi 171,2 g d'éthanamido-2 (nitro-4 phényl)-3 propanoate de méthyle (2S) sous forme d'un solide blanc cotoneux fondant à 126°C.

### Préparation de l'(amino-4 phényl)-3 éthanamido-2 propanoate de méthyle (2S):

A une suspension de 170,0 g d'éthanamido-2 (nitro-4 phényl)-3 propanoate de méthyle (2S), 417,5 g de zinc en poudre et 850 cm³ de méthanol à 10°C, sous agitation, est additionnée une solution de 341,6 g de chlorure d'ammonium dans 850 cm³ d'eau de telle sorte que la température du mélange soit maintenue inférieure à 25°C. A 25°C, la suspension est filtrée, rincée par 850 cm³ de méthanol puis rincée par 450 cm³ d'eau. Le filtrat et les deux lavages sont réunis, portés à reflux pour fractionner la totalité du méthanol puis ramené à température ambiante. A 20°C sont ajoutés 2000 cm³ de dichlorométhane. Le mélange est agité à 20°C pendant 30 minutes puis décanté. La phase inférieure organique est soutirée, séchée sur sulfate de sodium anhydre. Après filtration du sulfate de sodium, la solution est concentrée à 30°C sous pression réduite (20 kPa) puis séchée sous pression réduite (20 kPa) à environ 25°C.

On obtient ainsi 123,4 g d'(amino-4 phényl)-3 éthanamido-2 propanoate de méthyle (2S) sous forme d'un solide jaune fondant à 141°C.

### Préparation de l'éthanamido-2 (méthanamido-4 phényl)-3 propanoate de méthyle (2S):

A 158 cm³ d'anhydride acétique à 0°C sont additionnés goutte à goutte sous agitation 76,7 cm³ d'acide formique. La solution est ensuite chauffée à 50°C pendant 2 heures puis ramenée à 0°C, température à laquelle sont additionnés 150 cm³ de tétrahydrofuranne. A ce mélange est additionnée une solution de 148,6 g d'(amino-4 phényl)-3 éthanamido-2 propanoate de méthyle (2S) dans 1500 cm³ de tétrahydrofuranne de telle sorte que la température du mélange réactionnel soit maintenue inférieure à 10°C. En fin d'addition, on laisse le mélange revenir à 25°C, puis on ajoute 1500 cm³ d'eau. Le mélange est porté à reflux, le tétrahydrofuranne est fractionné puis le mélange est ramené à 25°C et maintenu à cette température pendant 3 heures. La suspension obtenue est filtrée, rincée par 150 cm³ d'eau, essorée, séchée sous pression réduite (20 kPa) à 40°C.

On obtient ainsi 123,5 g d'éthanamido-2 (méthanamido-4 phényl)-3 propanoate de méthyle (2S) sous forme d'un solide beige fondant à 126°C.

### Préparation de l'éthanamido-2 [(N-méthylamino)-4 phényl]-3 propanoate de méthyle (2S) :

A une solution de 100,0 g d'éthanamido-2 (méthanamido-4 phényl)-3 propanoate de méthyle (2S) dans 3000 cm³ de tétrahydrofuranne à 20°C sous agitation sont additionnés sous azote 378 cm³ d'une solution 2M de borane methyl sulfure dans le tétrahydrofuranne de telle sorte que la température du mélange réactionnel soit maintenue inférieure à 25°C. En fin d'addition, l'agitation du mélange réactionnel est maintenue 1 heure à 25°C puis 1000 cm³ de méthanol sont additionnés goutte à goutte. En fin d'addition du méthanol, le mélange est agité à 25°C sous azote pendant 16 heures puis concentré sous pression réduite (20 kPa) à une température de 40°C. En fin de concentration, on laisse revenir à 25°C sous azote puis 1000 cm³ de dichlorométhane et 1000 cm³ d'une solution aqueuse saturée en chlorure de sodium sont additionnés. Le mélange est agité 30 minutes à 25°C puis décanté. La phase organique inférieure est soutirée et séchée sur sulfate de sodium anhydre. Après filtration du sulfate de sodium, la solution est concentrée à 30°C sous pression réduite (20 kPa).

On obtient ainsi 95,1 g d'éthanamido-2 (N-méthylamino-4 phényl)-3 propanoate de méthyle (2S) sous forme d'un solide blanc fondant à 151°C.

### Préparation du dichlorhydrate de l'acide amino-2 ((N-méthylamino)-4 phényl)-3 propanoïque (2S) :

Une solution de 95,0 g d'éthanamido-2 (N-méthylamino-4 phényl)-3 propanoate de méthyle (2S) dans 285 cm³ d'eau et 570 cm³ d'acide chlorhydrique à 36 % est portée à reflux pendant 5 heures puis concentrée. Au mélange concentré refroidi à 80°C sont additionnés 900 cm³ de propanol-2. La suspension obtenue est maintenue à 80°C pendant 2 heures puis ramenée à température ambiante. Le précipité est filtré, rincé deux fois par 200 cm³ de propanol-2, essoré puis séché sous pression réduite (20 kPa) à environ 40°C.

On obtient ainsi 77,5 g de dichlorhydrate de l'acide amino-2 ((N-méthylamino)-4 phényl)-3 propanoïque (2S) sous forme d'un solide blanc fondant à 210°C.

## Revendications

1. Un procédé de préparation d'une forme énantiomère de l'acide amino-2 (alkylamino-4 phényl)-3 propanoïque de formule générale : 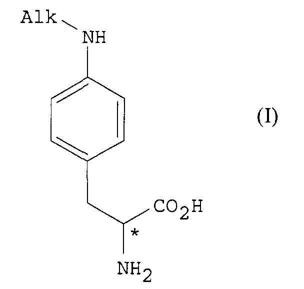 dans laquelle Alk représente un radical alcoyle contenant 1 à 2 atomes de carbone, sous forme (S) ou (R), ainsi que de ses sels, à partir respectivement de la (L)-phénylalanine ou de la (D)-phénylalanine, par nitration, puis transformation du radical nitro de la nitro-4 phénylalanine obtenue, de formule générale : 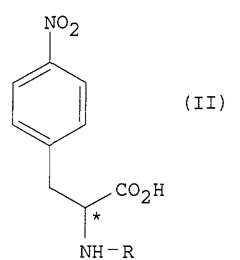 dans laquelle R est un atome d'hydrogène ou un radical protecteur, en un radical alcoylamino, après protection le cas échéant de la fonction amine de la phénylalanine.

## Patentansprüche

1. Verfahren zur Herstellung einer enantiomeren Form von 2-Amino-3-(4-alkylamino-phenyl)-propansäure der allgemeinen Formel (I) 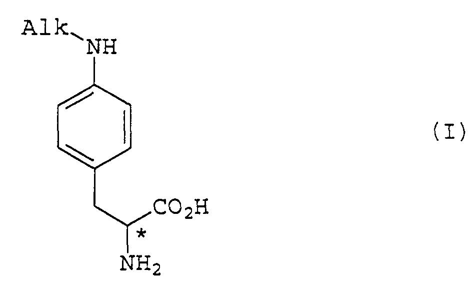 in der Alk einen Rest Alkyl mit 1 bis 2 Kohlenstoffatomen darstellt, in der Form (S) oder (R), sowie von ihren Salzen, jeweils ausgehend von (L)-Phenylalanin oder (D)-Phenylalanin durch Nitrierung, anschließende Umwandlung des Restes Nitro von dem erhaltenen 4-Nitro-phenylalanin der allgemeinen Formel (II) 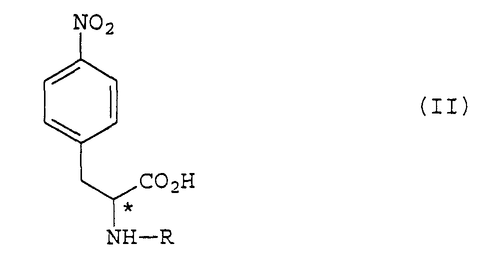 in der R ein Wasserstoffatom oder eine Schutzgruppe ist, in einen Rest Alkylamino, gegebenenfalls nach Schutz der Funktion Amino des Phenylalanins.

## Claims

1. Process for the preparation of one enantiomeric form of 2-amino-3-(4-alkylaminophenyl)propanoic acid of general formula: 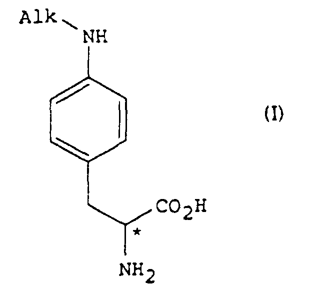 in which Alk represents an alkyl radical containing 1 to 2 carbon atoms, in (S) or (R) form, as well as the salts thereof, from (L)-phenylalanine or (D)-phenylalanine respectively, by nitration, and then conversion of the nitro radical of the 4-nitrophenylalanine obtained, of general formula: 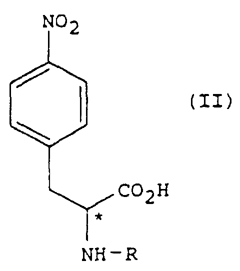 in which R is a hydrogen atom or a protecting radical, into an alkylamino radical, after protection, where necessary, of the amine function of the phenylalanine.
